# EUROPEAN PATENT APPLICATION

(11) **EP 4 018 952 A1**
(43) Date of publication of application: **29.06.2022**
(21) Application number: 21216328.1
(22) Date of filing: 21.12.2021
(51) Int. Cl.: A61B 18/14

(54) **BALLOON CATHETER WITH IRRIGATED TIP ELECTRODE**

(30) Priority: 22.12.2020 US 202017131571
(71) Applicant: Biosense Webster (Israel) Ltd, Yokneam, 2066717 (IL)
(72) Inventor: The designation of the inventor has not yet been filed
(74) Representative: Carpmaels & Ransford LLP

(57) **Abstract**

An apparatus includes a shaft, configured for insertion into a body of a subject, an inflatable balloon disposed over the shaft, a plurality of leaf electrodes coupled to the balloon and shaped to define one or more leaf-electrode apertures, a tip electrode disposed at a distal end of the balloon, coupled to a distal end of the shaft, and shaped to define one or more tip-electrode apertures, a first fluid-delivery tube passing through the shaft and configured to deliver a fluid into the balloon via at least one shaft aperture in the shaft, such that the fluid passes through the leaf-electrode apertures, and a second fluid-delivery tube passing through the shaft and configured to deliver the fluid to the tip electrode, such that the fluid passes through the tip-electrode apertures. Other embodiments are also described.

## Description

### FIELD OF THE INVENTION

The present invention is related to medical devices for the ablation of tissue.

### BACKGROUND

US Patent Application Publication 2008/0249463 describes a fluid delivery catheter configured to distribute fluid through each electrode by varying the diameter of a catheter lumen. Uniform or different fluid flow rates through longitudinally spaced apart elution holes may be achieved. Exemplary fluids for use with the catheter include a cooling fluid, a therapeutic fluid, and a medication.

### SUMMARY OF THE INVENTION

There is provided, in accordance with some embodiments of the present invention, an apparatus including a shaft, configured for insertion into a body of a subject, and an inflatable balloon disposed over the shaft. The apparatus further includes a plurality of leaf electrodes coupled to the balloon and shaped to define one or more leaf-electrode apertures. The apparatus further includes a tip electrode disposed at a distal end of the balloon, coupled to a distal end of the shaft, and shaped to define one or more tip-electrode apertures. The apparatus further includes a first fluid-delivery tube passing through the shaft and configured to deliver a fluid into the balloon via at least one shaft aperture in the shaft, such that the fluid passes through the leaf-electrode apertures. The apparatus further includes a second fluid-delivery tube passing through the shaft and configured to deliver the fluid to the tip electrode, such that the fluid passes through the tip-electrode apertures.

In some embodiments, the apparatus further includes a Luer hub connected to respective proximal ends of the first fluid-delivery tube and second fluid-delivery tube and configured to deliver the fluid, from a pump, to the first fluid-delivery tube and second fluid-delivery tube.

In some embodiments, the shaft includes:
a shaft body; and
a flow diverter coupled to, and extending distally from, the shaft body,
wherein the balloon is disposed over the shaft by virtue of being coupled to the flow diverter, and
wherein the flow diverter is shaped to define the shaft aperture.

There is further provided, in accordance with some embodiments of the present invention, a method including inserting a shaft into a body of a subject. The method further includes, subsequently to inserting the shaft, inflating an inflatable balloon disposed over the shaft. The method further includes, subsequently to inflating the balloon, ablating tissue of the subject by passing electrical current between (i) a plurality of leaf electrodes coupled to the balloon, and (ii) a tip electrode disposed at a distal end of the balloon and coupled to a distal end of the shaft, while fluid is delivered (a) into the balloon, via at least one shaft aperture in the shaft, by a first fluid-delivery tube passing through the shaft, such that the fluid passes into the body through leaf-electrode apertures in the leaf electrodes, and (b) to the tip electrode by a second fluid-delivery tube passing through the shaft, such that the fluid passes through tip-electrode apertures in the tip electrode.

In some embodiments, the fluid is delivered, from a pump, to the first fluid-delivery tube and second fluid-delivery tube by a Luer hub connected to respective proximal ends of the first fluid-delivery tube and second fluid-delivery tube.

In some embodiments, the shaft includes:
a shaft body; and
a flow diverter coupled to, and extending distally from, the shaft body,
the balloon being disposed over the shaft by virtue of being coupled to the flow diverter, and
the flow diverter being shaped to define the shaft aperture.

The present invention will be more fully understood from the following detailed description of embodiments thereof, taken together with the drawings, in which:

### BRIEF DESCRIPTION OF THE DRAWINGS

Fig. 1 is a schematic illustration of an ablation system, in accordance with some embodiments of the present invention;
Fig. 2A is a schematic illustration of the distal end of a probe, in accordance with some embodiments of the present invention;
Fig. 2B is a schematic illustration of a longitudinal cross section through the distal end of a probe, in accordance with some embodiments of the present invention; and
Fig. 3 is a schematic illustration of the proximal end of a probe, in accordance with some embodiments of the present invention.

### DETAILED DESCRIPTION OF EMBODIMENTS

### OVERVIEW

US Patent Application 16/863,815, whose disclosure is incorporated herein by reference, describes a balloon probe for use in ablation procedures. The probe comprises a plurality of leaf electrodes coupled to the surface of the balloon, along with a tip electrode at the distal end of the balloon. The tip electrode functions as the return (or "indifferent") electrode for the ablation currents, typically while the tip electrode presses against the ablated tissue. While the ablation is performed, an irrigating fluid is passed from the balloon through apertures in the leaf electrodes. The fluid transfers heat from the electrodes to the blood, and also dilutes the blood so as to inhibit the blood from coagulating or becoming charred.

A challenge, when using this probe, is that the tip electrode may overheat due to insufficient heat transfer therefrom.

To address this challenge, embodiments of the present invention provide additional apertures in the tip electrode, such that the irrigating fluid may flow through, and hence transfer additional heat from, the tip electrode. Typically, the irrigating fluid is transferred to the tip electrode via a separate fluid-delivery tube, such that the rate of fluid flow through the tip electrode remains relatively constant even while the tip electrode and/or leaf electrodes press against the tissue.

### SYSTEM DESCRIPTION

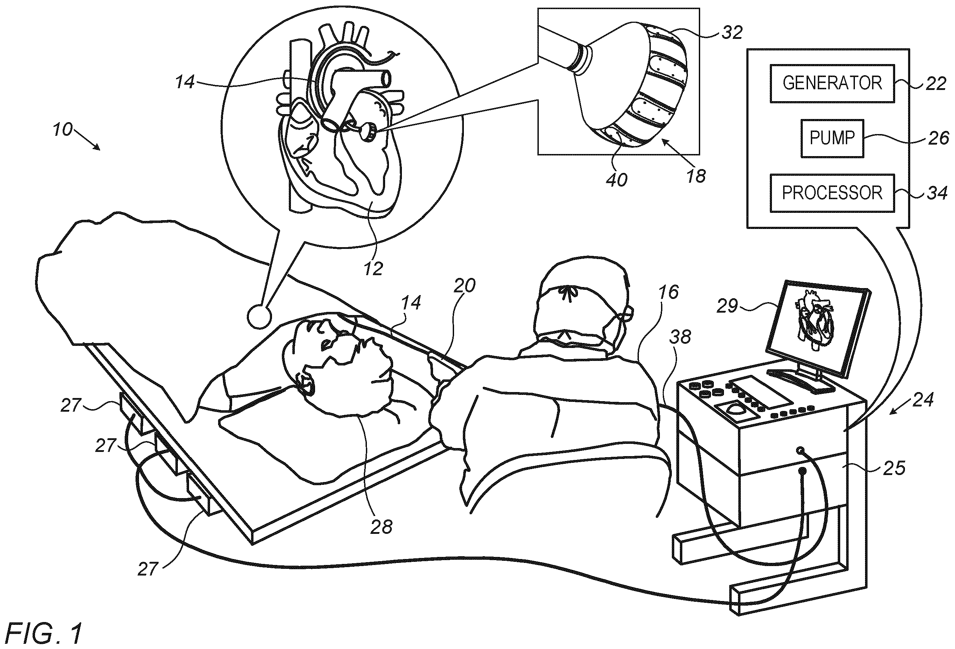

Reference is initially made to Fig. 1, which is a schematic illustration of an ablation system 10, in accordance with some embodiments of the present invention.

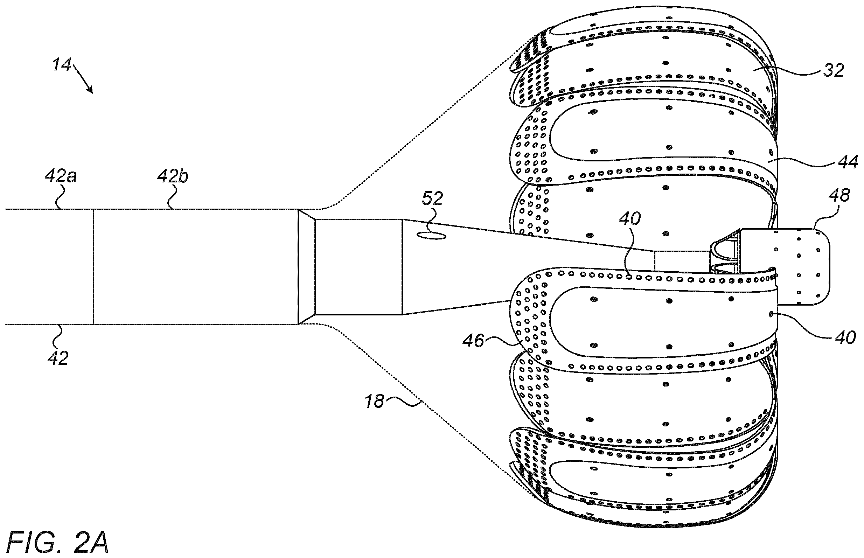

System 10 comprises an intrabody probe 14, configured for percutaneous insertion, by a physician 16, into the body of a subject 28. Probe 14 comprises an inflatable balloon 18 disposed at the distal end of the probe, a plurality of leaf electrodes 32 coupled to balloon 18, and a tip electrode 48 (Figs. 2A-B) disposed at the distal end of the balloon.

Following the insertion of probe 14, the probe is navigated, by the physician, to a target site within the subject's body, such as a chamber or vascular structure of the heart 12 of the subject. Subsequently, inflatable balloon 18 is inflated, by pumping an irrigating fluid (typically saline) through the balloon, as further described below with reference to Figs. 2A-B.

Subsequently, the balloon is brought into contact with abnormal tissue at the target site, such as abnormal endocardial tissue. The abnormal tissue is then ablated by passing electrical current between leaf electrodes 32 and the aforementioned tip electrode. In other words, the ablation is performed bipolarly, with the tip electrode functioning as a return electrode. (The ablation may include radiofrequency (RF) ablation, pulsed field ablation (PFA), or irreversible electroporation (IRE).) While the ablation is performed, the irrigating fluid is continually pumped into the body of the subject via apertures 40 in the distal end of the probe, thus keeping the balloon inflated, transferring heat from the electrodes to blood of the subject, and/or diluting the blood.

System 10 further comprises a signal generator 22, which is connected to the each of the aforementioned electrodes via wires running through probe 14. Signal generator 22 is configured to generate ablation signals for ablating tissue as described above. System 10 further comprises a pump 26, configured to pump the aforementioned irrigating fluid through probe 14 to the distal end of the probe.

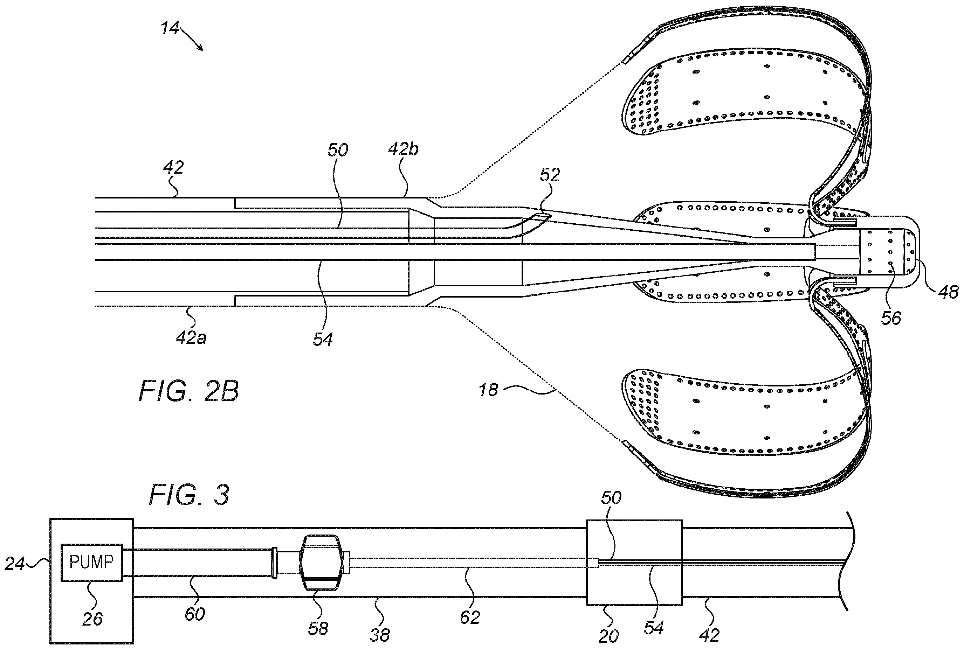

Typically, signal generator 22 and pump 26 are contained in a console 24. In some embodiments, the proximal end of probe 14 is connected directly to console 24. In other embodiments, the proximal end of probe 14 is connected to the console via a cable 38, which contains the aforementioned wires along with a main tube 60 (shown in Fig. 3) for delivering fluid from the pump. As further described below with reference to Fig. 3, cable 38 may further contain a Luer hub for distributing fluid from the pump.

Typically, probe 14 further comprises a handle 20, comprising suitable controls for positioning and orienting the distal end of the probe.

System 10 further comprises a processor 34, which is typically contained in console 24. Processor 34 is configured to control signal generator 22 and pump 26. The processor may be further configured to track the location of the probe as described immediately below, and/or to process any electrophysiological signals received from the electrodes.

In some embodiments, the distal end of the probe comprises an electromagnetic sensor, e.g., as described in co-assigned US Patent Application 16/863,815, whose disclosure is incorporated herein by reference. In such embodiments, system 10 may further comprise a plurality of magnetic field generators 27, along with another signal generator 25, which is typically contained in console 24. Signal generator 25 is configured to drive magnetic field generators 27 to generate a magnetic field. The magnetic field induces, in the electromagnetic sensor, a signal that varies with the location and orientation of the sensor. Processor 34 receives this signal, and, in response to the signal, tracks the location and orientation of the distal end of the probe, e.g., as described in US Patents 5,391,199, 5,443,489, and 6,788,967 to Ben-Haim, in US Patent 6,690,963 to Ben-Haim et al., in US Patent 5,558,091 to Acker et al., and in US Patent 6,177,792 to Govari, whose respective disclosures are incorporated herein by reference.

In other embodiments, the distal end of the probe is tracked using another type of tracking system. For example, electrical currents may be passed between electrodes 32 and a plurality of electrode patches coupled to the subject's body. Based on the distribution of the currents, the processor may look up the position of probe in a preconfigured position map, as described, for example, in co-assigned US Patents 7,536,218 and 8,456,182, whose respective disclosures are incorporated herein by reference.

Typically, system 10 further comprises a display 29. In response to tracking the probe, processor 34 may display, on display 29, an icon representing the distal end of the probe superimposed over a pre-acquired image of the target site, thus indicating the location of the probe to physician 16. Alternatively or additionally, the processor may display, on display 29, an electrophysiological map showing areas of electrical activation at the target site.

Typically, probe 14 further comprises one or more temperature sensors (e.g., thermocouples and/or thermistors) and/or pressure sensors, e.g., as described in US Patent Application 16/863,815. Signals from these sensors are carried over wires, which run through probe 14, to processor 34. Processor may process these signals and, in response thereto, display the sensed temperature and/or pressure on display 29.

System 10 may further comprise other components not shown in Fig. 1, such as an electrocardiogram (ECG) monitor, an imaging device, and/or interface circuitry, such as analog-to-digital (A/D) and digital-to-analog (D/A) circuitry, for interfacing between processor 34 and other components of system 10.

Reference is now made to Fig. 2A, which is a schematic illustration of the distal end of probe 14, in accordance with some embodiments of the present invention. Reference is further made to Fig. 2B, which is a schematic illustration of a longitudinal cross section through the distal end of probe 14, in accordance with some embodiments of the present invention.

Probe 14 comprises a shaft 42. As described above with reference to Fig. 1, the probe further comprises balloon 18, which is disposed over shaft 42 at the distal end of the probe. As described in US Patent Application 16/863,815, the balloon may have a relatively small diameter, such as a diameter less than 20 mm or 15 mm, when fully inflated. (Given that the balloon, typically, is not circular, the "diameter" of the balloon refers to the maximal transverse cross-sectional diameter of the balloon.)

The probe further comprises leaf electrodes 32, which are coupled to the balloon (in particular, the outer surface thereof), typically such that each leaf electrode runs along the surface of the balloon from the distal end of the balloon to the approximate axial center of the balloon. Typically, as shown in Figs. 2A-B, leaf electrodes 32 are evenly distributed around the circumference of the balloon. For example, the probe may comprise eight leaf electrodes, the respective centers of successive leaf electrodes being separated from one another by 45 degrees.

Typically, each leaf electrode comprises a conductive plating 44 disposed over a substrate 46 that is mounted to the balloon. Such an electrode may be manufactured using suitable printed circuit board (PCB) manufacturing technology known in the art. Advantageously, the leaf electrodes are sufficiently flexible so as not to crack when the balloon is inflated or deflated.

Apertures 40, mentioned above with reference to Fig. 1, may be formed in conductive plating 44 and/or substrate 46. Apertures 40 are aligned with corresponding apertures in the surface of the balloon (e.g., as described in US Patent 10,638,976, whose disclosure is incorporated herein by reference), such that irrigating fluid may pass through apertures 40 from the interior of the balloon. A first fluid-delivery tube 50, which passes through the shaft, is configured to deliver the fluid into the balloon via at least one aperture 52 in the shaft, such that the fluid may pass through apertures 40 into the body of the subject. Typically, aperture 52 is disposed in a side wall of the shaft.

Probe 14 further comprises a tip electrode 48 disposed at the distal end of the balloon and coupled to the distal end of the shaft. A second fluid-delivery tube 54, which passes through the shaft, is configured to deliver irrigating fluid to tip electrode 48, such that the fluid passes through one or more additional apertures 56 in the tip electrode.

In some embodiments, the shaft comprises a shaft body 42a and a flow diverter 42b coupled to, and extending distally from, shaft body 42a. For example, flow diverter 42b may be fitted over the distal end of the shaft body. Typically, the shaft body is made from a flexible polymer, while the flow diverter is made from a rigid material, such as polyether ether ketone (PEEK). Alternatively, the flow diverter may also be made from a flexible polymer, so as to allow deflection of tip electrode 48. In addition to carrying the distal end of second fluid-delivery tube 54, the flow diverter carries a wire (not shown) that electrically connects the tip electrode to generator 22 (Fig. 1).

In such embodiments, balloon 18 is disposed over the shaft by virtue of being coupled to the flow diverter, and the flow diverter is shaped to define aperture 52. Advantageously, this configuration may facilitate collapsing the balloon. It is noted that in the embodiment of Fig. 2A, the tip electrode 48 includes a cylindrical configuration in which the tip extends long the longitudinal axis beyond the periphery of the balloon for suitable tissue contact by tip electrode 48. In other words, a distal cross-section of the tip electrode extends further along the longitudinal axis as compared to any cross-section of the balloon.

Reference is now made to Fig. 3, which is a schematic illustration of the proximal end of the probe, in accordance with some embodiments of the present invention.

Typically, the probe further comprises a Luer hub 58, which is connected to the respective proximal ends of the first fluid-delivery tube and second fluid-delivery tube. Luer hub 58 is configured to deliver irrigating fluid from pump 26, via main tube 60, to the two fluid-delivery tubes. In some embodiments, the Luer hub is contained within cable 38, proximally to handle 20. (In such embodiments, a reinforcing tube 62 may reinforce the proximal portions of the first and second fluid-delivery tubes disposed within the cable.) In other embodiments, the Luer hub is contained within handle 20 or shaft 42.

In general, the respective flow rates through the fluid-delivery tubes may be controlled by varying the respective diameters of the tubes, along with the number and size of the apertures in the electrodes. Typically, the flow rate through the first fluid-delivery tube, which delivers fluid to the balloon, is 2-10 times greater than the flow rate through the second fluid-delivery tube, which delivers fluid to the tip electrode.

In other embodiments, two separate pumps deliver irrigating fluid to the two fluid-delivery tubes, respectively. In such embodiments, the respective fluid pressures in the tubes may differ from one another.

It will be appreciated by persons skilled in the art that the present invention is not limited to what has been particularly shown and described hereinabove. Rather, the scope of embodiments of the present invention includes both combinations and subcombinations of the various features described hereinabove, as well as variations and modifications thereof that are not in the prior art, which would occur to persons skilled in the art upon reading the foregoing description. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. Apparatus, comprising:
a shaft, configured for insertion into a body of a subject;
an inflatable balloon disposed over the shaft;
a plurality of leaf electrodes coupled to the balloon and shaped to define one or more leaf-electrode apertures;
a tip electrode disposed at a distal end of the balloon, coupled to a distal end of the shaft, and shaped to define one or more tip-electrode apertures;
a first fluid-delivery tube passing through the shaft and configured to deliver a fluid into the balloon via at least one shaft aperture in the shaft, such that the fluid passes through the leaf-electrode apertures; and
a second fluid-delivery tube passing through the shaft and configured to deliver the fluid to the tip electrode, such that the fluid passes through the tip-electrode apertures.

2. The apparatus according to claim 1, further comprising a Luer hub connected to respective proximal ends of the first fluid-delivery tube and second fluid-delivery tube and configured to deliver the fluid, from a pump, to the first fluid-delivery tube and second fluid-delivery tube.

3. The apparatus according to claim 1, wherein the shaft comprises:
a shaft body; and
a flow diverter coupled to, and extending distally from, the shaft body,
wherein the balloon is disposed over the shaft by virtue of being coupled to the flow diverter, and
wherein the flow diverter is shaped to define the shaft aperture.

4. A method, comprising:
inserting a shaft into a body of a subject;
subsequently to inserting the shaft, inflating an inflatable balloon disposed over the shaft; and
subsequently to inflating the balloon, ablating tissue of the subject by passing electrical current between (i) a plurality of leaf electrodes coupled to the balloon, and (ii) a tip electrode disposed at a distal end of the balloon and coupled to a distal end of the shaft, while fluid is delivered (a) into the balloon, via at least one shaft aperture in the shaft, by a first fluid-delivery tube passing through the shaft, such that the fluid passes into the body through leaf-electrode apertures in the leaf electrodes, and (b) to the tip electrode by a second fluid-delivery tube passing through the shaft, such that the fluid passes through tip-electrode apertures in the tip electrode.

5. The method according to claim 4, wherein the fluid is delivered, from a pump, to the first fluid-delivery tube and second fluid-delivery tube by a Luer hub connected to respective proximal ends of the first fluid-delivery tube and second fluid-delivery tube.

6. The method according to claim 4, wherein the shaft includes:
a shaft body; and
a flow diverter coupled to, and extending distally from, the shaft body,
wherein the balloon is disposed over the shaft by virtue of being coupled to the flow diverter, and
wherein the flow diverter is shaped to define the shaft aperture.
